(19) Europäisches Patentamt
European Patent Office
Office européen des brevets

(11) Publication number: 0 342 615 A1

# (12) EUROPEAN PATENT APPLICATION

(21) Application number: 89108793.4

(51) Int. Cl.4: C07D 301/14

(22) Date of filing: 16.05.89

(30) Priority: 17.05.88 IT 2059688

(43) Date of publication of application:
23.11.89 Bulletin 89/47

(84) Designated Contracting States:
AT BE CH DE ES FR GB LI NL

(71) Applicant: ISTITUTO GUIDO DONEGANI S.p.A.
Via Caduti del Lavoro
I-28100 Novara(IT)

(72) Inventor: Ricci, Marco
5, Via Brescia
I-28100 Novara(IT)
Inventor: Querci, Cecilia
10, Viale Volta
I-28100 Novara(IT)

(74) Representative: Barz, Peter, Dr. et al
Patentanwälte Dipl.-Ing. G. Dannenberg Dr.
P. Weinhold, Dr. D. Gudel Dipl.-Ing. S.
Schubert, Dr. P. Barz Siegfriedstrasse 8
D-8000 München 40(DE)

## (54) Process for the preparation of epoxides.

(57) A process for the preparation of epoxides by oxidation of the corresponding olefinic compounds is disclosed, said process being characterized in that the oxidation is carried out in a two-phase system which comprises:

(a) an organic phase which contains the olefinic substrate, catalytic amounts of a porphyrinic complex of manganese and, optionally, at least one co-catalytic component selected from:
(i) a nitrogen-containing, heterocyclic base; and
(ii) an "onium" salt,
all of said components being dissolved in an organic solvent substantially immiscible with water;

(b) an aqueous phase containing a solution of an alkali or alkaline earth metal salt of an organic peracid, preferably magnesium monoperoxyphthalate hexahydrate.

The resulting products may be used in the fields of foamed materials (polyurethanes), plasticizers and epoxy resins and as intermediates for fine chemistry.

EP 0 342 615 A1

## PROCESS FOR THE PREPARATION OF EPOXIDES

The present invention relates to a process for the preparation of epoxides by starting from olefinic compounds. More particularly, it relates to a process according to which the epoxides are prepared by oxidation of the corresponding olefinic compounds with an alkali or alkaline earth metal salt of an organic peracid, in a two-phase system comprising a liquid aqueous and a liquid organic phase and in the presence of suitable catalysts selected from porphyrinic complexes of manganese. The epoxides obtained by the process of the present invention may be used in several industrial fields, for example, in the fields of foamed materials (urethanes), plasticizers and epoxy resins but, above all, as intermediates for pharmaceutical, veterinary or plant protection products, etc.

Several processes for the direct conversion of olefinic compounds into the corresponding epoxides are known. The most widely-used process still is the epoxidation with organic peracids such as, for example, performic acid, peracetic acid, m-chloro-perbenzoic acid, etc. (J. March "Advanced Organic Chemistry", 3rd edition, J. Wiley & Sons, 1985, page 735). This method provides a considerable flexibility and simplicity of operation which makes it still preferred over less expensive processes such as the ones which use organic hydroperoxides or even hydrogen peroxide as oxidants.

The use of peracids is, however, not free of drawbacks with respect to both safety and the selectivities obtained in the epoxidation reactions. In fact, as regards the first aspect, peracids often have to be prepared by using highly concentrated solutions of hydrogen peroxide, which solutions are potentially dangerous. Furthermore, they generally are rather unstable and even m-chloro-perbenzoic acid, which is regarded as being one of the safest peracids, can explode and shows a certain impact sensitivity. Furthermore, as regards the selectivities of the epoxidations, many peracids, although being per se only weakly acidic, may be reduced to form the corresponding carboxylic acids, the latter being characterized by rather high acidity constants and, therefore, capable of reacting with the epoxide just formed, thus lowering the reaction yields.

These drawbacks were eliminated, in principle, by the use of magnesium monoperoxyphthalate hexahydrate as oxidant. Said salt may easily be prepared, is very stable and, due to its solubility in water and its capability of being employed in biphasic systems (aqueous and organic phase), can easily be kept separated from the formed epoxide. By using this salt, various olefinic compounds were converted into the corresponding epoxides with yields of from 50 to 98% (P. Brougham, M.S. Cooper, D.A. Cummerson, H. Heaney, N. Thompson, Synthesis 1987, 1015). The efficiency of conversion is, however, poor. In fact, the reactions require several hours (from 3 to 7 hours) even if being carried out at temperatures which are mostly higher than room temperature and usually range from 25 to 50 °C.

An object of the present invention is, therefore, to provide a novel, simple and efficient method for the oxidation of compounds containing olefinic unsaturations to yield the corresponding epoxides, particularly, a method which uses as oxidant an inexpensive, stable and readily available salt such as, for example, magnesium monoperoxyphthalate hexahydrate, advantageously activated by small amounts of a suitable catalyst.

Another object is the provision of a process for the oxidation of the above substrates which is particularly reliable as regards achievable productivity, the volumes and times employed being the same, with a sustantially quantitative selectivity.

It has, unexpectedly, been found that the above and other objects, which will become clearer from the following disclosure, are achieved according to the present invention by a process for the preparation of epoxides by means of the oxidation of the corresponding olefinic compounds, which process is characterized in that said olefinic compounds are oxidized in a two-phase system comprising:

a) an organic phase which contains the unsaturated olefinic compounds, catalytic amounts of at least one porphyrinic complex of manganese and, optionally, at least one co-catalytic component selected from
(i) nitrogen-containing heterocyclic bases; and
(ii) "onium" salts as defined below;
all of said components being dissolved in an organic solvent which is substantially immiscible with water;

(b) an aqueous phase comprising an aqueous solution of at least one alkali metal or alkaline earth metal salt of an organic peracid, preferably a solution of magnesium monoperoxyphthalate hexahydrate.

The use of catalytic amounts of porphyrinic complexes of manganese, optionally in combination with nitrogen-containing heterocyclic bases and/or "onium" salts, in the epoxidation of olefinic substrates with an alkali(ne) (earth) metal salt of a peracid, such as magnesium monoperoxyphthalate hexahydrate, makes it possible to carry out said reactions within a few minutes, even at temperatures lower than room temperature. This, in turn, allows the achievement of high selectivities with respect to the desired product,

even under conditions which result in a substantially quantitative conversion of the olefin or the olefinic compound. Consequently, no burdensome separation of the obtained epoxide from the unreacted substrate is necessary.

The epoxidation of the olefinic compounds or substrates, as disclosed herein, is preferably conducted under the following conditions.

As has already been mentioned, the reaction is carried out in a biphasic system (aqueous liquid/organic liquid) under strong stirring, i.e., stirring which allows both phases to be in continuous and efficient contact with each other.

Operating temperatures and pressures are determined by the reactivity and the nature of the substrate, in particular by its volatility. In practice, the reaction is preferably carried out at temperatures ranging from the freezing point of the aqueous phase up to $+40^{\circ}C$ and preferably from $0^{\circ}C$ to about $+25^{\circ}C$.

As regards pressure, the present process is usually carried out under atmospheric pressure but, in the case of particularly volatile substrates. application of higher pressures, generally within the range of from 1 to about 50 atm, may be desirable.

The reaction time depends on the nature and the amounts of the porphyrinic complex and the heterocyclic base used as well as on the nature of the substrate and the selected temperature. Times within the range of from a few seconds up to one hour are generally sufficient for completing the reaction, even at $0^{\circ}C$.

The compounds containing olefinic unsaturations, which can be subjected to the epoxidation process according to the present invention, are preferably represented by formula (I):

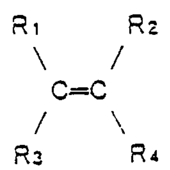

(I)

wherein $R_1$, $R_2$, $R_3$ and $R_4$, the same or different from each other, may optionally contain functional groups inert under the reaction conditions, and represent hydrogen atoms or hydrocarbyl groups, which may, possibly, be branched such as alkyl and alkenyl groups containing up to 30, preferably 1 to 12 and, particularly, 1 to 8 carbon atoms, cycloalkyl and cycloalkenyl groups containing from 3 to 12, preferably from 5 to 8 carbon atoms and aryl, alkylaryl and alkenylaryl groups containing up to 12, preferably 6 to 10 carbon atoms. Furthermore, any two of the $R_1$, $R_2$, $R_3$ and $R_4$ groups may be combined to form a ring containing up to 12, preferably 5 to 8 carbon atoms and having one or more (preferably 1 to 3) unsaturations.

Examples of the groups just mentioned are:

| | |
|---|---|
| alkyl: | methyl, ethyl, n- and i-propyl,n-, i-, sek- and tert-butyl, pentyl, hexyl, octyl, decyl; |
| alkenyl: | vinyl, 1- and 2-propenyl, 1- and 2-butenyl; |
| cycloalkyl: | cyclopentyl,cyclohexyl, cyclooctyl; |
| cycloalkenyl: | cyclopentenyl, cyclohexenyl, cyclooctenyl; |
| aryl: | phenyl, naphthyl, biphenylyl, anthracenyl. |

Preferably at least one and, particularly, at least two of the groups $R_1$, $R_2$, $R_3$ and $R_4$ represent hydrogen and only one or two of the remaining groups carry a functional group.

Substituent groups inert under the reaction conditions are, for example, F, Cl, Br, I and nitro, hydroxy (preferably $C_{1-6}$) alkoxy, carbonyl, carboxy ester (e.g. acetate), amide, nitrile etc.

As mentioned above, the $R_1$, $R_2$, $R_3$ and $R_4$ groups can also be alkenyl groups. In other words, the process according to the present invention can also be applied to substrates containing more than one olefinic unsaturation such as, for example either conjugated or non-conjugated dienes and trienes.

Examples of (poly)olefinic compounds suitable for use in the present invention comprise unsaturated hydrocarbons such as propylene, the butenes (1-butene, 2-butene, i-butene), pentenes (for example 1-pentene, 2-pentene, i-pentene), 1-dodecene and, in general, linear or branched mono- and di-olefins containing up to 30 (preferably from 2 to 18) carbon atoms, cyclohexene, cyclooctene, limonene, styrene, α-methyl-styrene, the stilbenes, unsaturated alkyl halides (for example the chlorides, bromides and iodides) such as the allyl halides and the methallyl halides (particularly the chlorides and bromides), unsaturated

acids and esters such as 3-butenoic acid or oleic acid and their esters (particularly their $C_{1-8}$ esters), etc.

In the epoxidation of the above substrates porphyrine complexes of manganese are used as catalysts.

These complexes comprise the class of the meso(tetraaryl)porphyrinates and can be represented by the formula:

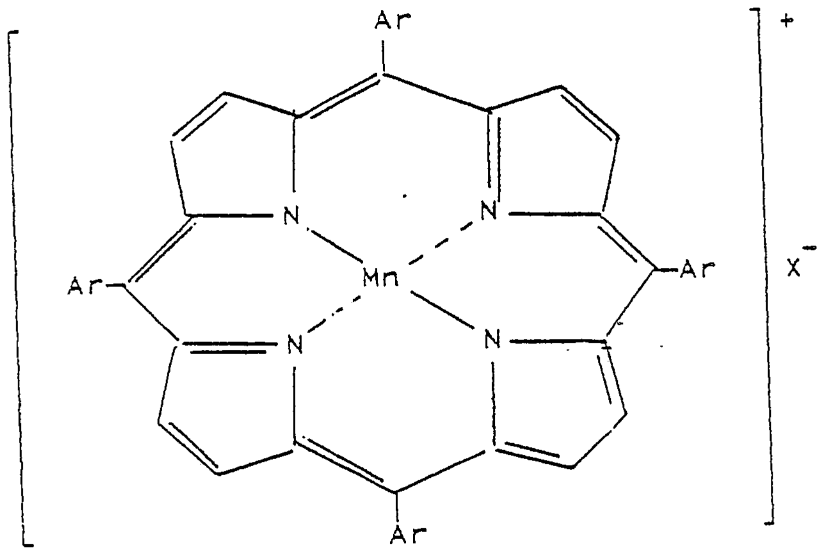

(II)

wherein:

| | |
|---|---|
| X | represents a monovalent anion stable under the reaction conditions such as, for example, chloride or acetate, and |
| Ar | represents aryl groups such as phenyl, naphthyl and anthracenyl, which may be the same or different from each other, optionally substituted with groups which are inert under the reaction conditions. |

In particular, the aryl group may carry one or more substituents at least one of them being preferably selected from halogen atoms such as chlorine, bromine, fluorine, the lower ($C_{1-6}$, preferably $C_{1-4}$) alkyl and alkoxy groups (for example, methyl, ethyl, propyl, butyl and the corresponding alkoxy groups), nitro, sulfonyl, amino, amido etc.

Preferably Ar is selected from phenyl, 2,6-dichlorophenyl, mesityl, perfluorophenyl, 2-fluorophenyl, etc. However, due to its stability, which is much higher than that of the other related compounds, the use of the complex in which Ar represents 2,6-dichlorophenyl is preferred.

The porphyrinic complex is normally employed in amounts of from 0.0001 to 0.01 mole, preferably from 0.0005 to 0.005 mole per mole of substrate.

In order to increase the reaction efficiency, co-catalysts can be employed together with the porphyrinic complexes of manganese used as the catalyst in the process of the present invention. Said co-catalysts are preferably selected from at least one of

1. nitrogen-containing heterocyclic bases; and/or
2. as phase-transfer agents, "onium" salts.

In the most preferred embodiment of the process according to the present invention both types of the above co-catalytic compounds are present, i.e.,:

1. at least one nitrogen-containing heterocyclic base; and
2. at least one "onium" salt.

The nitrogen-containing heterocyclic bases preferably have from 5 to 7 ring atoms, 1 or 2 of which being nitrogen atoms, the remainder being carbon atoms. Some (for example 1 or 2) of said carbon atoms can be replaced by heteroatoms, such as O and S.

As bases, pyridines or imidazoles can be used to particular advantage. Preferably at least one base selected from pyridine, 4-picoline, 3-phenyl-pyridine, 4-tert-butyl-pyridine, 1-hexyl-imidazole, etc. is used.

The heterocyclic base is used in amounts which can vary within a wide range, for example, within the range of from 0.0001 to 1 mole per mole of substrate, although amounts ranging from 0.0001 to 0.5 mole per mole of substrate are regarded as being preferred.

The "onium" salts which can suitably be used as phase transfer agents preferably are the well-known quaternary salts represented by the general formula (III):

$R_5R_6R_7R_8M^+Y^-$ (III)

wherein:

| | |
|---|---|
| M | represents a pentavalent element selected from N and P; |
| Y | represents a stable, inorganic cation such as chloride or bisulfate; |
| $R_5$, $R_6$, $R_7$ and $R_8$, | the same or different from each other, represent monovalent alkyl or alkylaryl groups containing a total of up to 70, preferably of from 20 to 40, carbon atoms. |

Examples of groups $R_5$, $R_6$, $R_7$ and $R_8$ are methyl, ethyl, propyl, butyl, pentyl, hexyl, heptyl, octyl, nonyl, decyl, undecyl, dodecyl, tetradecyl, hexadecyl, octadecyl, benzyl and phenethyl.

Examples of "onium" salts which may advantageously be used according to the present invention are benzyl-dimethyl-hexadecyl-ammonium chloride and dimethyl-[di-octadecyl(75%) + di-hexadecyl(25%)]-ammonium chloride. This latter is known under the trade name Arquad$^{(R)}$2HT (manufactured by AKZO-Chemie, Italy).

The amount of "onium" salt is not critical for the present process but preferably ranges from 0.001 to 0.02 mole, particularly from 0.001 to 0.01 mole per mole of substrate.

In general, an inert solvent substantially immiscible with water, is used as the organic solvent. The chlorinated hydrocarbons such as dichloromethane, dichloroethane, trichloroethane, etc. as well as mixtures thereof are particularly suitable.

The amount of solvent used is not critical but preferably is such that the concentration of the substrate in the organic phase is within the range of from 10 to about 95% by weight. Higher or lower concentrations may, however, also be employed even if they sometimes are not very practical. The solvent can also be composed of the olefinic substrate to be epoxidized.

As oxidant any alkali metal or alkaline earth metal salt of any organic peracid (percarboxylic acid) can be used.

However, the use of the Na, K and Mg salts of mono-peroxy-phthalic acid, the Mg salts of monoperoxy-hexahydrophthalic, monoperoxy-maleic and monoperoxy-citraconic acid and their mixtures has been found to be particularly effective.

Owing to its stability and its availability at low prices, magnesium monoperoxy-phthalate hexahydrate, known as H 48 (manufactured by INTEROX, United Kingdom), is particularly preferred. The amount of oxidant can also be varied within a wide range, for example, from 0.1 to 10 moles per mole of olefinic substrate.

Since, however, the reaction is advantageously carried out until the conversion of the substrate is substantially complete (in order to avoid a burdensome and expensive separation of the obtained epoxide from the unreacted substrate) a certain excess of oxidant, i.e., an amount of from 2 to 5 moles per mole of olefinic substrate, is preferably employed.

The concentration of the oxidant in the aqueous phase may, for example, be within the range of from 1% to the value at which the aqueous solution is saturated.

Concentrations close to the saturation concentration are preferred since they allow the achievement of a higher productivity and, thus, are more favourable from an economic point of view.

At the end of the reaction, after the separation of the phases, the epoxide formed (which is dissolved in the organic phase) can be isolated and purified according to conventional techniques, for example, by distillaton or column chromatography.

The following examples are given to illustrate the present invention.

## EXAMPLE 1

A 25 ml flask equipped with magnetic stirrer was charged with 2.5 ml of methylene chloride, 0.416 g of styrene (4 mmole), 5 mg of 5,10,15,20-tetra(2,6-dichlorophenyl)porphyrinate of manganese acetate (5 µmole), 20 µl of 4-picoline (0.2 mmole) and 4.1 mg of benzyl-dimethyl-hexadecyl-ammonium chloride (10

µmole).

The above mixture was cooled to 0 °C, whereafter a solution, previously filtered and also kept at 0 °C, of 4.4 g of magnesium monoperoxyphthalate hexahydrate (90%, 8 mmole) in 16 ml of water was added. The resulting mixture was vigorously stirred for 30 seconds, whereupon the two phases were separated and the organic phase was subjected to gas-chromatographic analysis, using n-dodecane as the internal standard. Said analysis revealed a styrene conversion of 100%, with a selectivity to styrene oxide of 90%.

EXAMPLE 2

The reaction was carried out in the same way as in example 1 but the amounts of styrene, magnesium monoperoxyphthalate hexahydrate and water were doubled and, hence, a molar ratio of substrate to catalyst of 1,600 was used. After 60 seconds of reaction at 0 °C the gas-chromatographic analysis of the organic phase indicated a styrene conversion of 100% with a selectivity to styrene oxide of 80%.

EXAMPLE 3

The reaction was carried out in the same way as in example 1, except that styrene was replaced by 0.44 g of cyclooctene (4 mmole). After 3 minutes of reaction at 0 °C, the gas-chromatographic analysis of the organic phase revealed a cyclooctene conversion of 100% with a selectivity to epoxy-cyclooctane of 93%.

EXAMPLE 4

The reaction was carried out in the same way as in example 3 but with the amounts of cyclooctene, magnesium monoperoxyphthalate hexahydrate and water being doubled and, hence, with a molar ratio of substrate to catalyst of 1,600. After 3 minutes of reaction at 0 °C the gas-chromatographic analysis of the organic phase indicated a cyclooctene conversion of 100% with a selectivity to epoxy-cyclooctane of 85%.

EXAMPLE 5

The reaction was carried out in the same way as in example 1, except that styrene was replaced by 0.336 g (2 mmole) of dodecene and 2.2 g of magnesium monoperoxyphthalate hexahydrate (90%) dissolved in 8 ml of water, were used. After 8 minutes of reaction at 0 °C, the gas-chromatographic analysis of the organic phase (with n-octadecane as the internal standard) indicated a conversion of 1-dodecene of 100% with a selectivity to 1,2-epoxy-dodecane of 96%.

EXAMPLE 6

The reaction was carried out in the same way as in example 1 except that styrene was replaced by 0.071 g (0.5 mmole) of isobutyl vinyl-acetate and 0.55 g of magnesium monoperoxyphthalate hexahydrate (90%), dissolved in 2 ml of water, were used. After 30 minutes of reaction at 0 °C the gas-chromatographic analysis of the organic phase revealed a conversion of isobutyl vinylacetate of 85% with a selectivity to isobutyl 3,4-epoxy-butyrate of 98%.

EXAMPLE 7

The reaction was carried out in the same way as in example 3 but without 4-picoline. After 3 minutes of reaction at 0 °C the gas-chromatographic analysis of the organic phase indicated a conversion of cyclooctene of 65% with a selectivity to epoxy-cyclooctane of 94%.

EXAMPLE 8

The reaction was carried out in the same way as in example 3 but without benzyl-dimethyl-hexadecyl-ammonium chloride. After 3 minutes of reaction at 0 °C the gas-chromatographic analysis of the organic phase indicated a conversion of cyclooctene of 54%, with a selectivity to epoxy-cyclooctane of 45%.

EXAMPLE 9 (Comparative Example)

The reaction was carried out in the same way as in example 3 but in the absence of 5,10,15,20-tetra-(2,6-dichlorophenyl)porphyrinate of manganese acetate. After 3 minutes of reaction at 0 °C the gas-chromatographic analysis of the organic phase showed that the conversion of cyclooctene was 42% with a selectivity to epoxy-cyclooctane of 12%.

EXAMPLE 10

The reaction was carried out in the same way as in example 1 but with the amounts of styrene (0.104 g, 1 mmole) of magnesium monoperoxyphthalate hexahydrate (1.1 g at 90%, 2 mmole) and water (4 ml) decreased, and with 5,10,15,20-tetra(2,6-dichlorophenyl)porphyrinate of manganese acetate replaced by 5,10,15,20-tetraphenylporphyrinate of manganese chloride (3.5 mg, 5 µmole). After 2 minutes of reaction at 0 °C, the gas-chromatographic analysis of the organic phase showed that the conversion of styrene was 67% with a selectivity to styrene oxide of 57%.

EXAMPLE 11

The reaction was carried out in the same way as in example 3 but replacing 4-picoline by pyridine (20 µl, 0.25 mmole). After 3 minutes of reaction at 0 °C the gas-chromatographic analysis of the organic phase showed that the cyclooctene conversion was 100% with a selectivity to epoxy-cyclooctane of 92%.

EXAMPLE 12

The reaction was carried out in the same way as in example 3 but in the absence of both 4-picoline and benzyl-dimethyl-hexadecyl-ammonium chloride. After 3 minutes of reaction at 0 °C the gas-chromatographic analysis of the organic phase indicated a conversion of cyclooctene of 32% with a selectivity to epoxy-cyclooctane of 25%.

## Claims

Process for the preparation of epoxides by oxidation of the corresponding olefinic compounds, characterized in that said olefinic compounds are oxidized in a two-phase system comprising:

(a) an organic phase which comprises said olefinic compound and catalytic amounts of at least one porphyrinic complex of manganese, both components being dissolved in an organic solvent substantially immiscible with water;

(b) an aqueous phase comprising a solution of at least one alkali(ne) (earth) metal salt of an organic peracid.

2. Process according to claim 1, characterized in that the organic phase contains, dissolved in said organic solvent, at least one co-catalytic component selected from:

(a) nitrogen-containing heterocyclic bases; and

(b) "onium" salts;

and, preferably, contains both types of components (a) and (b).

3. Process according to any one of claims 1 and 2, characterized in that the porphyrinic manganese complex catalyst comprises at least one meso(tetraaryl)porphyrinate of formula (II):

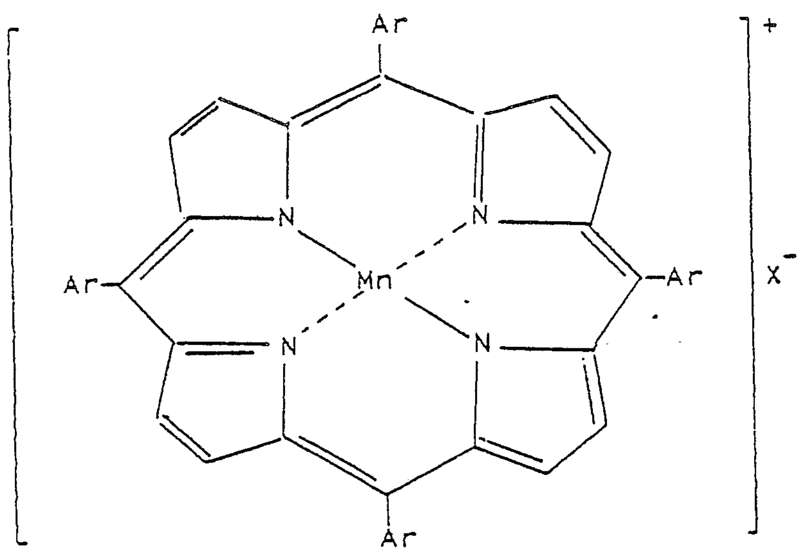

(II)

wherein:

X represents a monovalent anion stable under the reaction conditions, preferably selected from chloride and acetate, and the

radicals Ar represent optionally substituted aryl groups, the same or different from each other, preferably selected from phenyl, naphthyl and anthracenyl groups, optionally substituted with at least one group selected from lower alkyl and lower alkoxy, nitro, sulfonyl, amino, amido, Cl, F and Br.

4. Process according to claim 3, characterized in that, in said porphyrinic complex of manganese of formula (II), Ar is selected from phenyl, 2-fluoro-phenyl, mesityl, perfluorophenyl and, preferably, 2,6-dichlorophenyl.

5. Process according to any one of claims 2 to 4, characterized in that said nitrogen-containing heterocyclic base is selected from the pyridines and the imidazoles and, preferably, comprises at least one of pyridine, 4-picoline, 3-phenyl-pyridine, 4-tert-butyl-pyridine and 1-hexyl-imidazole.

6. Process according to any one of claims 2 to 5, characterized in that the "onium" salt is selected from quaternary salts of formula (III):

$R_5R_6R_7R_8M^+Y^-$ (III)

wherein:

| | |
|---|---|
| M | is N or P; |
| Y | represents a stable, inorganic anion preferably selected from chloride or bisulfate; |
| $R_5$, $R_6$, $R_7$ and $R_8$, | the same or different from each other, represent monovalent alkyl groups or alkylaryl groups containing a total of up to 70, preferably from 20 to 40 carbon atoms; |

and preferably is composed of benzyl-dimethyl-hexadecyl-ammonium chloride and/or dimethyl-[di-octadecyl(75%) + di-hexadecyl(25%)]-ammonium chloride.

7. Process according to any one of claims 1 to 6, characterized in that said alkali(ne) (earth) metal salt of the organic peracid is selected from the Na, K and Mg salts of mono-peroxy-phthalic acid, the Mg salts of monoperoxy-hexahydro-phthalic acid, monoperoxy-maleic acid and monoperoxy-citraconic acid and mixtures of said salts, and preferably is magnesium monoperoxyphthalate hexahydrate.

8. Process according to any one of claims 1 to 7, characterized in that the olefinic compound is selected from compounds having the formula (I):

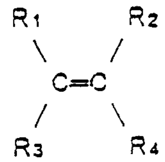

(I)

wherein $R_1$, $R_2$, $R_3$ and $R_4$, which optionally may contain functional groups inert under the reaction conditions, are the same or different from each other and represent hydrogen or linear or branched hydrocarbyl groups selected from alkyl groups and alkenyl groups containing up to 30 carbon atoms, cycloalkyl groups and cycloalkenyl groups containing from 3 to 12 carbon atoms, aryl, alkylaryl or alkenylaryl groups containing up to 12 carbon atoms; and any two of the above groups $R_1$, $R_2$, $R_3$ and $R_4$ may be combined to form a ring containing up to 12 carbon atoms and having one or more unsaturations.

9. Process according to claim 8, characterized in that the olefinic compound is a compound of formula (I) which contains at least one group selected from nitro, hydroxy, alkoxy, carbonyl, carboxy, ester, amide, nitrile and the halogen atoms.

10. Process according to any one of claims 1 to 9, characterized in that the olefinic compound is selected from propylene, the butenes, the pentenes, 1-dodecene, cyclohexene, cyclooctene, limonene, styrene, α-methyl-styrene, the stilbenes, the allyl halides and the methallyl halides, 3-butenoic acid or oleic acid and their esters or is a polyunsaturated olefin preferably selected from dienes and trienes.

11. Process according to any one of the preceding claims, characterized in that it is carried out at temperatures ranging from the freezing point of the aqueous phase to +40° C and, preferably, from 0° C to about +25° C.

12. Process according to any one of the preceding claims, characterized in that it is carried out under a pressure ranging from atmospheric pressure to about 50 atm.

13. Process according to any one of the preceding claims, characterized in that the porphyrinic complex is used in amounts of from 0.0001 to 0.01 mole, preferably from 0.0005 to 0.005 mole, per mole of olefinic substrate.

14. Process according to any one of the preceding claims, characterized in that said alkali(ne) (earth) metal salt of the organic peracid is used in amounts of from 0.1 to 10 moles, preferably from 2 to 5 moles per mole of olefinic substrate.

15. Process according to any one of claims 2 to 14, characterized in that said nitrogen-containing heterocyclic base is used in amounts of from 0.0001 to 1 mole, preferably from 0.0001 to 0.5 mole per mole of olefinic substrate.

16. Process according to any one of claims 2 to 15, characterized in that said "onium" salt is used in amounts of from 0.001 to 0.02 mole, preferably from to 0.01 mole per mole of olefinic substrate.

17. Process according to any one of the preceding claims, characterized in that the inert organic solvent substantially immiscible with water is selected from chlorinated hydrocarbons, preferably dichloromethane, dichloroethane, trichloroethane and mixtures thereof, and the olefinic compound used as starting material.

18. Process according to any one of the preceding claims, characterized in that the concentration of the olefinic substrate in the organic phase ranges from 10% to about 95% by weight.

19. Process according to any one of the preceding claims, characterized in that the concentration of the alkali(ne) (earth) metal salt of the organic peracid in the aqueous phase is at least 1% by weight and, preferably, corresponds substantially to the saturation concentration of the salt in the aqueous phase.

European Patent Office

# EUROPEAN SEARCH REPORT

Application Number

EP 89 10 8793

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | SYNTHESIS, no. 11, November 1987, pages 1015-1017, Stuttgart, DE; P. BROUGHAM et al.: "Oxidation reactions using magnesium monoperphthalate: A comparison with m-chloroperoxybenzoic acid" * Whole article * | 1-19 | C 07 D 301/14 |
| Y | JOURNAL OF THE AMERICAN CHEMICAL SOCIETY, vol. 106, no: 22, 31st October 1984, pages 6668-6676, American Chemical Society, Gaston, US; B. MEUNIER et al.: "Sodium hypochlorite: A convenient oxygen source for olefin epoxidation catalyzed by (porphyrinato)manganese complexes" * Whole article * | 1-19 | |
| | | | TECHNICAL FIELDS SEARCHED (Int. Cl.4) |
| | | | C 07 D 301/00<br>C 07 D 303/00 |

The present search report has been drawn up for all claims

| Place of search | Date of completion of the search | Examiner |
|---|---|---|
| THE HAGUE | 02-08-1989 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)